# EUROPÄISCHE PATENTSCHRIFT

(11) **EP 2 753 277 B1**
(45) Veröffentlichungstag und Bekanntmachung des Hinweises auf die Patenterteilung: **09.06.2021**
(21) Anmeldenummer: 12759082.6
(22) Anmeldetag: 06.09.2012
(51) Int. Cl.: A61F 13/02

(54) **SCHLAUFENSCHLITZER**
LOOP SLITTER
DISPOSITIF D'INCISION DE BOUCLE

(30) Priorität: 09.09.2011 DE 102011082458
(43) Veröffentlichungstag der Anmeldung: 16.07.2014
(73) Patentinhaber: Luye Pharma AG, 83714 Miesbach (DE)
(72) Erfinder: GRADER, Ludwig, 56626 Andernach (DE); PIOTROWSKI, Holger, 83727 Schliersee (DE)
(74) Vertreter: Zehetner Patentanwälte
(86) Internationale Anmeldenummer: PCT/EP2012/067450
(87) Internationale Veröffentlichungsnummer: WO 2013/034658

(56) Entgegenhaltungen:
- US-A- 3 574 026
- US-A- 4 610 189
- US-A- 4 867 821
- US-A1- 2007 074 809
- US-B1- 6 187 128

## Beschreibung

Die vorliegende Erfindung bezieht sich auf Vorrichtungen zur Herstellung transdermaler therapeutischer Systeme (TTS) mit mehrteiligen Schutzfolien.

Transdermale therapeutische Systeme umfassen gewöhnlich ein Wirkstoffdepot, das auf einer Seite von einer wirkstoffundurchlässigen Rückschicht und auf der dieser gegenüberliegenden Applikationsseite von einer Schutzfolie ganzflächig bedeckt ist. Die Schutzfolie wird vor einer Applikation des Systems entfernt und ist, um ihr Abziehen im Applikationsfall zu vereinfachen, meist mehrteilig und vorzugsweise zweiteilig ausgeführt, wobei sich die einzelnen Schutzfolienteile überlappen oder an der Trennstelle stumpf aneinander anschließen können.

Gegenwärtig sind zwei Grundtypen transdermaler therapeutischer Systeme bekannt, Matrixsysteme und Reservoirsysteme. Bei den sogenannten Matrixsystemen ist der Wirkstoff in einer Polymermatrix enthalten, die zumeist aus einem selbsthaftenden druckempfindlichen Polymer gebildet ist. Die Wirkstoffabgabe wird bei diesen Systemen ausschließlich über das Konzentrationsgefälle zur Haut gesteuert. Bei den sogenannten Reservoirsystemen ist der Wirkstoff in einem flüssigen, halbfesten oder festen Reservoir enthalten, wobei die Wirkstoffabgabe mittels einer Membran reguliert wird, die sich in der Regel an der zur Schutzfolie weisenden Applikationsseite des Wirkstoffdepots befindet.

Zur Herstellung transdermaler therapeutischer Systeme wird zunächst eine bahnförmige, je nach Art des herzustellenden TTS ein- oder mehrlagige Wirkstoffdepotschicht auf eine ebenfalls bahnförmige Trägerfolie aufgebracht und mit einer wiederum bahnförmigen Deckfolie abgedeckt. Eine der beiden Folien, d. h. Träger- oder Deckfolie, fungiert dabei als Rückschicht für die aus der Laminatbahn hergestellten transdermalen therapeutischen Systeme. Mit dem Begriff Laminatbahn wird in dieser Schrift allgemein ein Verbund bahnförmiger Schichten bezeichnet.

Um die transdermalen therapeutischen Systeme mit einer mehrteiligen Schutzfolie zu versehen, wird bei vielen Herstellungsverfahren in die andere der beiden Folien ein Schlitz eingebracht, wozu die Folie entweder gestanzt oder geschnitten wird. Der Schlitz muss sich hierbei vollständig durch die Schutzfolie hindurch erstrecken, damit die einzelnen Schutzfolienteile zur Applikation eines TTS unabhängig voneinander abgezogen werden können. Eine perforierte Ausführung des Schlitzes, bei der einzelne Schlitzabschnitte durch Stege voneinander getrennt sind, ist möglich. Da sich die Folie beim Einbringen des Schlitzes jedoch in Kontakt mit der Wirkstoffdepotschicht befindet, wird diese häufig mitgeschlitzt, wodurch sich an der Schlitzvorrichtung Wirkstoffe anlagern können, die in der Folge zu einer nicht zulässigen Kontamination der Schutzfolienaußenseite im Schlitzbereich führen können. Weist die Wirkstoffdepotschicht eine Membran an oder nahe der Applikationsseite auf, so besteht auch die Gefahr, dass diese Membran beim Einbringen des Schlitzes mit der eventuellen Folge einer unkontrollierten Wirkstoffabgabe verletzt wird.

Daher wird die andere der beiden Folien bei anderen Herstellungsverfahren von der Wirkstoffdepotschicht abgezogen und durch eine entsprechend mehrteilige Schutzfolienbahn ersetzt. Aufgrund des Kontakts mit der Wirkstoffdepotschicht ist die abgezogene Folie kontaminiert und kann zum Schutze von Personen und Umwelt nicht mehr weiter verwendet werden. Stattdessen ist sie kostenaufwändig zu entsorgen.

Um diese Kosten zu vermeiden wird in der Patentschrift DE 44 05 296 C1 ein Herstellungsverfahren beschrieben, bei dem die andere der beiden Folien zwar zunächst ebenfalls von der Wirkstoffdepotschicht abgezogen, dann aber in einer eigenen Produktionsschlaufe geschlitzt und anschließend wieder auf die nach dem Abziehen der Folie freiliegende Oberfläche der Wirkstoffdepotschicht aufgebracht wird. Die Trennung der Schutzfolie vom restlichen Teillaminat aus Wirkstoffdepotschicht und Rückschichtfolie erfolgt über eine Umlenkwalze, wodurch sowohl die Schutzfolie als auch das Teillaminat jeweils einer Zugspannung ausgesetzt werden. Da Rückschichtfolien zum Erzielen eines hohen Tragekomforts der transdermalen therapeutischen Systeme überwiegend elastisch und damit dehnbar ausgebildet sind, während Schutzfolien zum besseren Abziehen meist nur flexibel, aber nicht elastisch sind, wird in diesen Fallgestaltungen das Teillaminat zwischen Abziehen und Wiederaufbringen der Schutzfolie stärker gedehnt als die Schutzfolie. Hierdurch entsteht eine Spannung zwischen Teillaminat und darauf aufgebrachter Schutzfolie, die zu einer Rolleffekt genannten Verbiegung bzw. einem Einrollen daraus hergestellter transdermaler therapeutischer Systeme führt und deren Handhabung erschwert.

In der US 2007/0074809 wird eine Vorrichtung zum Auftrennen einer aus einer mit einer Schutzfolie kaschierten Etikettenmaterialbahn bestehenden Laminatbahn, zum schneidenden Ausbilden von Etiketten in der Etikettenlaminatbahn und zum anschließenden Wiederaufkaschieren der Schutzfolie auf die Etikettenlaminatbahn beschrieben. Die Auftrennung erfolgt mit einer Klinge, wonach die Schutzfolie der Laminatbahn über eine Führungswalze in den Nip eines nachgeordneten Wiederaufkaschierwalzenpaars geführt wird, während die Etikettenmaterialbahn zunächst über eine Ambosswalze geführt und an dieser mit Hilfe einer Schneidwalze geschnitten wird, bevor sie dem Nip zwischen den nachgeordneten Wiederaufkaschierwalzen zugeführt wird. Zwischen der Auftrennklinge und der Führungswalze bzw. der Ambosswalze als auch zwischen diesen Walzen und dem Wiederaufkaschierwalzenpaar werden die jeweiligen Teile der Laminatbahn frei durch den Raum geführt.

In der US 6,187,128 B1 wird eine Etikettenmaterialbahn an einer Führungswalze von einer Laminatbahn abgetrennt und nach Durchlaufen einer Strecke im freien Raum über eine Vakuumambosswalze geführt, an der mit Hilfe einer Schneideinrichtung Etiketten in der Etikettenmaterialbahn ausgebildet werden. Die nach dem Abtrennen über eine Spannwalze geführte Abdeckfolienbahn wird nach dem Schneiden der Etikettenmaterialbahn in einen Nip zwischen der Vakuumambosswalze und einer Andruckwalze geführt und dort auf die geschnittene Etikettenmaterialbahn aufkaschiert. Die Etikettenmaterialbahn und die Abdeckfolienbahn werden nach dem Auftrennen jeweils durch den freien Raum geführt.

Es ist daher wünschenswert, transdermale therapeutische Systeme mit mehrteiliger Schutzfolie so herzustellen, dass keine der Folien der ursprünglichen Laminatbahn geopfert und ein wie zuvor beschriebener Rolleffekt vermieden wird.

Hierfür geeignete Herstellungsanlagen umfassen Ausführungsformen einer Vorrichtung nach Anspruch 1 zum Schlitzen einer einen Teil einer Laminatbahn (1) bildenden Folienbahn (2), wobei die Vorrichtung (10) eine Fördereinrichtung (11), eine Auftrenneinrichtung (12), eine Aufkaschiereinrichtung (15), eine Umlenkeinrichtung (13) und eine Schlitzvorrichtung (14) aufweist, dadurch gekennzeichnet, dass sowohl die Auftrenneinrichtung (12) als auch die Aufkaschiereinrichtung (15) an der Fördereinrichtung (11) anliegen oder einen Abstand zu dieser aufweisen, der kleiner oder gleich der Dicke der Laminatbahn (1) ist, die Aufkaschiereinrichtung (15) zur Auftrenneinrichtung (12) beabstandet und bezüglich der Förderrichtung der Fördereinrichtung (11) hinter der Auftrenneinrichtung (12) angeordnet ist, die Umlenkeinrichtung (13) bezüglich der Förderrichtung der Fördereinrichtung (11) zwischen der Auftrenneinrichtung (12) und der Aufkaschiereinrichtung (15) und in einem Abstand zur Fördereinrichtung (11) angeordnet ist, und die Schlitzvorrichtung (14) zum Einbringen eines Schlitzes in die von dem Laminat (1) getrennte Folienbahn (2) ausgebildet und im Bereich der Folienbahnführung zwischen Auftrenneinrichtung (12) und Aufkaschiereinrichtung (15) angeordnet ist, sodass die Folienbahn (2) an der Auftrenneinrichtung (12) von der Laminatbahn (1) getrennt und über die Umlenkeinrichtung (13) der Aufkaschiereinrichtung (15) zugeführt werden kann, während die den anderen Teil der Laminatbahn (1) bildende Teillaminatbahn (5) mit gleicher Geschwindigkeit an der Fördereinrichtung (11) anliegend zur Aufkaschiereinrichtung (15) geführt werden kann, und ein Schlitzen der Folienbahn (2) im Bereich der Folienbahnführung zwischen Auftrenneinrichtung (12) und Aufkaschiereinrichtung (15) erfolgen kann.

Zur Herstellung von wie oben als wünschenswert bezeichneter transdermaler therapeutischer Systeme eignet sich ferner ein Verfahrer nach Anspruch 15 zum Schlitzen einer einen Teil einer Laminatbahn bildenden Folienbahn, das auf einer Ausführungsform einer Vorrichtung nach Anspruch 1 ausgeführt wird und Schritte umfasst zum Trennen der Folienbahn von der restlichen Teilbahn der Laminatbahn an der Auftrenneinrichtung, zum Führen der abgetrennten Folienbahn von der Auftrenneinrichtung über die Umlenkeinrichtung zur Aufkaschiereinrichtung, zum Schlitzen der Folienbahn im Bereich zwischen Auftrenneinrichtung und Aufkaschiereinrichtung, und zum Führen der restlichen Teilbahn der Laminatbahn an der Fördereinrichtung anliegend von der Auftrenneinrichtung zur Aufkaschiereinrichtung, wobei die Teilbahn und die Folienbahn mit gleicher Geschwindigkeit von der Auftrenneinrichtung zur Aufkaschiereinrichtung transportiert werden.

Das beschriebene Verfahren und die beschriebene Vorrichtung transportieren den zwischen der Auftrenneinrichtung und der Aufkaschiereinrichtung nicht mehr von der Folienbahn bedeckten elastischen Teil der Laminatbahn an der Fördereinrichtung anliegend und somit von dieser gestützt entlang, so dass auf diesen elastischen Teil der Laminatbahn keine Kräfte ausgeübt werden, die zu einer Dehnung führen könnten. Ein wie zuvor beschriebener Rolleffekt wird somit wirksam vermieden.

Bei bevorzugten Ausführungsformen einer wie oben angegebenen Vorrichtung zum Schlitzen einer separat vom Rest der Laminatbahn, im Folgenden als Teillaminatbahn bezeichnet, geführten Folienbahn ist die Fördereinrichtung als Walze ausgebildet, wodurch eine stabile Transportoberfläche für die Förderung der an der Auftrenneinrichtung von der Folienbahn getrennten Teillaminatbahn gebildet wird. Bei anderen Ausführungsformen kann die Fördereinrichtung auch von einem Förderband gebildet sein, das im Förderbereich zur Stabilisierung vorzugsweise hinterstützt ist.

Bei Ausführung der Fördereinrichtung als Walze ist diese zweckmäßig zum Erzeugen eines Unterdrucks an zumindest dem von der Teillaminatbahn umschlungenen Bereich der Mantelfläche ausgebildet. Selbstverständlich kann auch bei Ausführung der Fördereinrichtung in Form eines Förderbands dieses zum Erzeugen eines Unterdrucks im Förderbereich ausgebildet sein. Durch den Unterdruck im Förderbereich ist sichergestellt, dass es beim Transport der Teillaminatbahn von der Auftrenneinrichtung zur Aufkaschiereinrichtung zu keinem Verzug der Teillaminatbahn relativ zur Fördereinrichtung kommen kann. Zusätzlich oder gegebenenfalls alternativ ist die Fördereinrichtung vorzugsweise zum Erzeugen eines Überdrucks an zumindest dem Bereich ausgebildet, der an den bezüglich der Förderrichtung hinteren Rand des von der Teillaminatbahn umschlungenen Bereichs der Mantelfläche der Walze anschließt. Bei Verwendung eines Förderbands als Fördereinrichtung schließt der entsprechende Überdruckbereich an den bezüglich der Förderrichtung hinteren Rand des zum Fördern der Teillaminatbahn ausgebildeten Bereichs an. Durch den Überdruck wird die Abgabe der Teillaminatbahn von der Fördereinrichtung unterstützt bzw. ein zu Dehnungen führendes Anhaften an dieser über den Förderbereich hinaus vermieden.

Bei bevorzugten Ausführungsformen ist wenigstens eine der zur separaten Führung der Folienbahn vorgesehenen Einrichtungen als Walze ausgeführt. Insbesondere ist wenigstens die Auftrenneinrichtung und/oder die Aufkaschiereinrichtung und/oder die Umlenkeinrichtung von einer Walze gebildet. Die Führung der Laminatbahn bzw. der Folienbahn über eine Walze ermöglicht einen Transport der jeweiligen Bahn, ohne dass diese relativ zu der sie stützenden Oberfläche verschoben wird. Materialabrieb und eventuell auf die Bahnen einwirkende Dehnungskräfte sind daher bei Führung über Walzen vorteilhaft minimiert. Bei besonders bevorzugten Ausführungsformen sind alle drei der genannten Einrichtungen von Walzen gebildet.

Bei anderen Ausführungsformen ist wenigstens eine der zur separaten Führung der Folienbahn vorgesehenen Einrichtungen in Form einer Klinge ausgebildet. Insbesondere ist wenigstens die Auftrenneinrichtung und/oder die Aufkaschiereinrichtung und/oder die Umlenkeinrichtung von einer Klinge gebildet. Klingen weisen gegenüber Walzen den Vorteil eines kleineren Umlenkradius auf, wodurch insbesondere bei Verwendung als Auftrenneinrichtung das Auftrennen der Laminatbahn in Teillaminatbahn und Folienbahn erleichtert wird.

Vorzugsweise ist die Umlenkeinrichtung bei Ausführungsformen sowohl in einem Abstand zur Auftrenneinrichtung als auch zur Aufkaschiereinrichtung angeordnet, wodurch eine vom Abstand zwischen Auftrenneinrichtung und Aufkaschiereinrichtung unabhängig lange Folienbahnschlaufe geschaffen wird und freie Abschnitte der Folienbahn geschaffen werden, an denen Einrichtungen zu deren Bearbeitung wie z. B. ein weiter unten näher erläuterter Stanzschlitzer, ein Drucker oder ein Lasergravurgerät angeordnet werden können. Um eine straffe Führung der Folienbahn in diesem Schlaufenbereich sicherzustellen und eine eventuelle Faltenbildung zu unterbinden, ist die Umlenkeinrichtung bei Ausführungsformen vorteilhaft so gelagert, dass sie auf die um sie geführte Folienbahn eine Zugspannung ausübt.

Bevorzugte Ausführungsformen weisen eine Schlitzvorrichtung auf, die einen Rotationskörper mit einer oder mehreren Schneiden umfasst, von denen jede entlang einer in Umfangsrichtung des Rotationskörpers geschlossenen Linie angeordnet und entweder von einem Abschnitt oder von mehreren entlang der Linie zueinander beabstandeten Abschnitten gebildet ist. Hiermit lässt sich mit einem kontinuierlich ablaufenden Schnittvorgang ein Schlitz mit sich periodisch wiederholender Kontur, eventuell in perforierter Weise, in die Folienbahn einbringen. Zur Ausbildung eines sich in Laufrichtung der Folienbahn geradlinig erstreckenden Schlitzes ist die Schnittkante der Schneide bei vorteilhaften Ausgestaltungen hiervon vorzugsweise kreisförmig ausgebildet. Zur Ausbildung eines zweidimensionalen linienförmigen Schlitzes, wie zum Beispiel eines schlangen- bzw. wellenförmigen Schlitzes, weist die Schnittkante der Schneide bei anderen vorteilhaften Ausgestaltungen zweckmäßig Abschnitte auf, die sich in Richtung der Rotationsachse des Rotationskörpers erstreckende Komponenten aufweist. Weitere Ausführungsformen weisen eine Schlitzvorrichtung auf, die eine mit einer Schneide versehene stehende Klinge umfasst. Bei Ausführungsformen kann die Schneide so angeordnet sein, dass sie zum sicheren Durchtrennen der Folienbahn die Umlenkeinrichtung am von der Folienbahn umschlungenen Bereich berührt. Bei stehenden oder kreisförmig ausgebildeten Schneiden greift die Schneide bei weiterhin vorteilhaften Ausgestaltungen vorzugsweise in eine am Umschlingungsbereich der Umlenkeinrichtung ausgebildete Nut ein. Bei diskontinuierlicher Bandführung oder komplexen Schlitzgeometrien, beispielsweise mit sich weit quer zur Bandlänge erstreckenden Komponenten oder zur Ausbildung von durch Perforierungen unterbrochenen Strukturen, ist die Schlitzvorrichtung vorteilhaft als zwischen Auftrenneinrichtung und Umlenkeinrichtung bzw. als zwischen Umlenkeinrichtung und Aufkaschiereinrichtung angeordnete Stanzvorrichtung ausgebildet, wobei hierzu eine Ausbildung als Hubstanze besonders bevorzugt wird.

Weitere Merkmale der Erfindung ergeben sich aus der nachfolgenden Beschreibung von Ausführungsbeispielen in Verbindung mit den Ansprüchen sowie den beiliegenden Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können bei erfindungsgemäßen Ausführungsformen die bei den nachstehend erläuterten Ausführungsbeispielen angeführten Merkmale in von den Beispielen abweichender Anzahl und Kombination verwirklicht sein. Bei der nachfolgenden Erläuterung einiger Ausführungsbeispiele der Erfindung wird auf die beiliegenden Figuren Bezug genommen, von denen
- Figur 1: den Aufbau einer Laminatbahn zur Herstellung transdermaler therapeutischer Systeme in einer schematischen Darstellung zeigt, und
- Figur 2: eine Prinzipskizze einer Ausführungsform einer Einrichtung zur separaten Bearbeitung einer von einer Laminatbahn abgetrennten Folienbahn in einer Produktionsschlaufe zeigt.

In den Figuren werden gleiche oder ähnliche Bezugszeichen für funktionell gleichwertige oder ähnliche Charakteristiken unabhängig von speziellen Ausführungsformen verwendet.

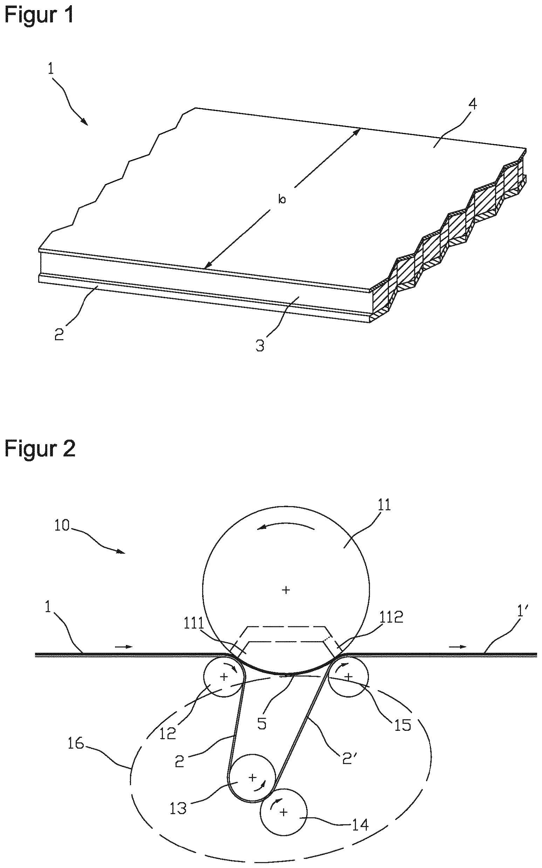

Figur 1 veranschaulicht den Aufbau einer Laminatbahn 1 zur Herstellung transdermaler therapeutischer Systeme mit mehrteiliger Schutzfolie in einer im Hinblick auf eine klare Darstellung des Sachverhalts nicht maßstabsgetreuen Prinzipdarstellung. Die Laminatbahn 1, von der in Figur 1 nur ein Teilstück gezeigt ist, weist eine bestimmte Breite b und eine im Verhältnis zur Breite wesentlich größere Länge auf. Die Laminatbahn 1 besteht aus drei Schichten, einer Trägerfolie 2, einer darauf aufgebrachten Wirkstoffdepotschicht 3 und einer auf die Wirkstoffdepotschicht aufgebrachten Deckfolie 4. Die Wirkstoffdepotschicht 3 kann sich je nach Anwendungsfall über die gesamte Breite b der Laminatbahn 1 oder wie im in Figur 1 vorgestellten Beispiel über nur einen Teil von deren Breite erstrecken. Bei vielen Anwendungen wird die Wirkstoffdepotschicht 3 statt wie gezeigt als Einzelbahn in Form mehrerer Bahnen auf die Trägerfolie 2 aufgebracht, wobei die einzelnen Bahnen in einem Abstand zueinander parallel verlaufen. Ferner kann die Wirkstoffdepotschicht 3 einlagig oder mehrlagig aufgebaut sein, wobei in letzterem Fall eine der Lagen von einer Membran gebildet sein kann.

Die Breite der Laminatbahn richtet sich nach dem jeweils herzustellenden transdermalen therapeutischen System und den Gegebenheiten der zur Herstellung jeweils verwendeten Apparatur. Üblich sind Breiten aus dem Bereich von etwa 25 bis 350 mm. Die Länge der Laminatbahn ist nicht festgelegt und wird in der Regel durch die verfügbaren Längen der zu ihrer Herstellung verwendeten Folienbahnen sowie der Menge der daraus herzustellenden transdermalen therapeutischen Systeme bestimmt. Längen von mehreren hundert Metern sind dabei durchaus üblich.

Bei dem in Figur 1 dargestellten Beispiel dient die Deckfolie 4 zur Ausbildung der wirkstoffundurchlässigen Rückschicht, die Trägerschicht 2 zur Ausbildung der Schutzfolie für aus der Laminatbahn 1 hergestellte transdermale therapeutische Systeme. Anders als zuvor dargelegt, kann die Wirkstoffdepotschicht selbstverständlich auch zuerst auf eine für die Ausbildung einer Rückschicht geeignete Trägerfolie 2 aufgebracht und anschließend mit einer zur Ausbildung der Schutzfolie ausgelegten Deckfolie 4 kaschiert werden.

Als zur Ausbildung von Rückschichten geeignete Folien können Polyolefine, insbesondere Polyethylen, Polyester und Polyurethane genannt werden.

Geeignete Materialien für die Schutzfolie sind beispielsweise Polyester, Polypropylen, Polyvinylchlorid, Aluminium und Papier, wobei zumindest eine Seite der Schutzfolie vorteilhaft eine Siliconbeschichtung, Polyethylenbeschichtung, Fluorsiliconbeschichtung oder Polytetrafluorethylenbeschichtung aufweist.

Für die Herstellung der Wirkstoffdepotschicht eignen sich grundsätzlich alle Substanzen und insbesondere Polymere, die bei der Herstellung von transdermalen Systemen eingesetzt werden und physiologisch unbedenklich sind, wie z. B. Homo- und Copolymere von (Meth)acrylaten, Polyvinylether, Polyisobutylene, Polyisoprenkautschuke, Styrol-Butadien-Copolymere oder Styrol-Butadien-StyrolCopolymere und Silicone. Beispiele für Wirkstoffe, die für die verschiedensten Anwendungen von TTS geeignet sind, sind etwa Analgetika, Antiemetika, Hormone, Antihistaminika, Antidepressiva und Betablocker.

Eine Schlaufenschlitzvorrichtung zur getrennten Bearbeitung einer Folienbahn eines Laminats unter Schonung des restlichen Laminats weist eine Auftrenneinrichtung 12 auf, an der die Laminatbahn 1 zunächst in die Folienbahn 2 und in eine verbleibende Teillaminatbahn 5 aufgetrennt wird (die Bezugszeichen beziehen sich auf die Darstellung von Figur 2). Die Folienbahn 2 wird nach der Auftrenneinrichtung 12 in einer über eine Umlenkeinrichtung 13 geführte Schlaufe zur Aufkaschiereinrichtung 15 geleitet, an der die im Bereich der Schlaufe bearbeitete Folienbahn 2' schließlich wieder auf die Teillaminatbahn 5 aufkaschiert wird. Zwischen der Auftrenneinrichtung 12 und der Aufkaschiereinrichtung 15 ist eine Fördereinrichtung 11 angeordnet, an der die Teillaminatbahn 5 anliegend von der Auftrenneinrichtung 12 zur Aufkaschiereinrichtung 15 befördert wird. Die Bearbeitung der Folienbahn 5 zum Einbringen eines Schlitzes erfolgt mithilfe einer Schlitzvorrichtung 14, die entlang der Schlaufe angeordnet ist.

Figur 2 zeigt eine schematische Darstellung einer Ausführungsform einer Schlaufenschlitzvorrichtung 10, bei der keine Zugkräfte auf den nicht der Bearbeitungsschlaufe geführten Teil 5 der Laminatbahn 1 ausgeübt werden. Die Lage der Bearbeitungsschlaufe ist in Figur 2 mithilfe eines mit einer gestrichelten Linie 16 umgrenzten Bereichs gekennzeichnet.

Die Schlaufenschlitzvorrichtung 10 umfasst in der dargestellten Ausführungsform eine Auflagewalze 11, eine Trennwalze 12, eine Schlaufenwalze 13, eine Schneidwalze 14 und eine Kaschierwalze 15. Die Laufrichtungen von Laminatbahn 1 und bearbeiteter Laminatbahn 1' sind in Figur 2 ebenso wie die Rotationsrichtungen der Walzen durch entsprechende Pfeile gekennzeichnet. Die Lage der Rotationsachsen der Walzen ist durch kleine Kreuze veranschaulicht.

Im zwischen Auflagewalze 11 und Trennwalze 12 ausgebildeten ersten Nip wird die Trägerfolie 2 von der zugeführten Laminatbahn 1 abgezogen, in einer die Schlaufenwalze 13 teilweise umrundenden Schlaufe geführt, an der Schlaufenwalze 13 von der Schneidwalze 14 geschlitzt und schließlich im zwischen Auflagewalze 11 und Kaschierwalze 15 gebildeten zweiten Nip wieder auf die Applikationsseite der Wirkstoffdepotschicht aufgebracht. Der die Wirkstoffdepotschicht 3 umfassende restliche Teil 5 der Laminatbahn 1 wird entlang der Auflagewalze 11 direkt vom ersten zum zweiten Nip geführt. Unter Nip ist in dieser Schrift und in Übereinstimmung mit dem üblichen Sprachgebrauch der Bereich zwischen zwei Walzen zu verstehen, in dem diese entweder direkt oder vermittelt über ein dazwischen befindliches Medium aufeinander einwirken bzw. Kräfte aufeinander ausüben. Die Existenz eines Nips setzt voraus, dass die Walzen entweder sich oder ein dazwischen befindliches Medium berühren.

Am ersten Nip wird die Laminatbahn 1 aufgetrennt, indem die Trägerfolie 2 am Ende des Nips entlang der Trennwalze 12 geführt wird, während der verbleibende Teil 5 der Laminatbahn 1 weiterhin an der Auflagewalze 11 anliegt. Die Rotationsgeschwindigkeiten beider Walzen 11 und 12 stehen dabei vorzugsweise im umgekehrten Verhältnis zu ihren Durchmessern, so dass die Laminatbahn 1 an ihren beiden Seiten mit derselben Geschwindigkeit in den ersten Nip gezogen und deren aufgetrennte Teilbahnen 2 und 5 den ersten Nip mit identischer Geschwindigkeit verlassen. Die Abstimmung der Umdrehungsgeschwindigkeiten von Auflagewalze 11 und Trennwalze 12 kann direkt durch entsprechende Abstimmung deren jeweiliger Antriebe, oder indirekt erfolgen, indem nur eine der beiden Walzen, vorzugsweise die Auflagewalze 11, angetrieben wird, während die andere Walze passiv durch die zwischen den Walzen geführte Laminatbahn 1 in Rotation versetzt wird.

Die Schlaufenwalze 13 ist zur Ausbildung einer ausreichenden Schlaufenlänge zweckmäßig in einem Abstand zur Trennwalze 12 angeordnet. Bei Folienbahnen 2, die von zur Faltenbildung neigenden Trägerfolien gebildet sind, kann die Schlaufenwalze zudem so gelagert sein, beispielsweise unter Verwendung einer hydraulischen Aufhängung, dass sie auf die Folienbahn 2 eine zusätzlich Zugspannung ausübt. Damit eine auf die Folienbahn 2 wirkende Zugspannung nicht bis zur Trennstelle der Laminatbahn 1 am Ende des ersten Nips zurückwirkt und sich dort auf den anderen Teil 5 der Laminatbahn 1 übertragen kann, wird die Trägerfolie 2 nach dem ersten Nip vorzugsweise noch über einen Teilumfang der Trennwalze 12 zum Ableiten der Zugspannung von der Trägerfolie 2 auf die Trennwalze geführt.

Die Schlaufenwalze 13 dient in der in Figur 2 dargestellten Ausführungsform einerseits als Umlenkrolle für die abgetrennte Trägerfolie 2 zur Definition der separaten Produktionsschlaufe, andererseits als Gegenlager für die Schneidwalze 14. Die Schneidwalze 14 bringt in die Trägerfolie einen in deren Längsrichtung verlaufenden Schlitz so ein, dass die Trägerfolie 2 nach Durchlaufen der von der Umlenkwalze 13 und der Schneidwalze 14 gebildeten Schlitzeinrichtung als mehrteilige Folienbahn 2' vorliegt.

Im einfachsten Fall weist die Schneidwalze 14 ein oder mehrere zueinander beabstandete Kreismesser mit umlaufenden Schneiden auf. Für einen Scherenschnitt liegen die Schneiden an den Seitenwänden von am Umfang der Umlenkwalze 13 ausgebildeten Nuten an. Hier sind nur gerade Schlitze möglich.

Insbesondere bei nicht geradlinig in Längsrichtung der Trägerfolie verlaufenden Schlitzen, z. B. bei schlangen- bzw. wellenförmigen Schlitzverläufen, ist die Oberfläche der Schneidwalze 14 vorzugsweise mit einer Schneidenkontur ausgestattet, die gegen die glatte Oberfläche der Umlenkwalze 13 drückt und so in die Trägerfolie 2 einen Schlitz mit einer der Schneidenkontur entsprechenden Verlaufsform einbringt. Bei Verwendung einer solchen als Quetschmesser bezeichneten Einrichtung rotiert die Schneidwalze 14 wie in Figur 2 dargestellt gleichlaufend zur Umlenkwalze 13, worunter zu verstehen ist, dass sich die Oberflächen beider Walzen an der Berührungsstelle in die selbe Richtung bewegen, die Umlaufrichtung beider Walzen jedoch gegensinnig ist.

Zum Einstellen einer bestimmten auf die Trägerfolie 2 wirkenden Zugspannung, die nicht allein durch die Bahnspannung bedingt ist, ist die Einheit aus Umlenkwalze 13 und Schneidwalze 14 vorzugsweise verschiebbar gelagert, wobei die Einstellung der Zugkraft über Gewichte, Federn, pneumatische oder hydraulische Lagerung erfolgen kann. Bei ausreichend steifen Trägerfolienbahnen 2 ist eine solche Zugspannung nicht unbedingt erforderlich. In diesen Fällen kann die Umlenkwalze 13 ohne Berührung der Teillaminatbahn 5 direkt an der auf der Trennwalze 12 aufliegenden Trägerfolienbahn und eventuell auch auf der auf der Kaschierwalze 15 aufliegenden geschlitzten Trägerfolienbahn 2' anliegen.

Die erläuterten Schlitzeinrichtungen eignen sich zum kontinuierlichen Schlitzen der Trägerfolienbahn 2. Bei diskontinuierlichen Schlitzverfahren, beispielsweise bei Verwendung von Stanzern, wie sie in der oben zitierten Patentschrift DE 44 05 296 C1 beschrieben sind, ist die Schlitzeinrichtung 14 vorzugsweise im von der Folienbahn 2 zwischen Trennwalze 12 und Schlaufenwalze 13 bzw. zwischen Schlaufenwalze 13 und Kaschierwalze 15 durchlaufenen Bereich angeordnet. Bei kontinuierlichem Vortrieb der Laminatbahn 1 bzw. 1' kann die Stanzvorrichtung, beispielsweise ein Hubstanzer, während eines jeden Stanzvorgangs mit der Folienbahn mitgeführt werden. Bei Verwendung einer ortsfesten Stanzeinrichtung erfolgt der Vortrieb der Laminatbahn zweckmäßig diskontinuierlich.

Erster und zweiter Nip der in Figur 2 veranschaulichten Schlaufenschlitzvorrichtung 10 sind beide so an der Auflagewalze 11 angeordnet, dass der die Deckschicht 4 und die Wirkstoffdepotschicht 3 umfassende Teil 5 der Laminatbahn über einen Teilumfang der Auflagewalze 11 an dieser aufliegend vom ersten zum zweiten Nip transportiert wird. Diese Anordnung stellt sicher, dass auf das Teillaminat 5 beim Transport vom ersten zum zweiten Nip keine zu einer Dehnung führende Zugkraft ausgeübt wird, wodurch der eingangs beschriebene Rolleffekt wirksam vermieden wird. Ein eventuelles Übertragen von auf die geschlitzte Trägerfolie 2' einwirkenden Zugkräften auf das Teillaminat 5 wird ähnlich wie bei der Trennvorrichtung am ersten Nip vermieden, indem die geschlitzte Trägerfolie vor Erreichen des zweiten Nips über einen Teilumfang der Kaschierwalze 15 an dieser anliegend geführt wird.

Insbesondere bei höheren Führungsgeschwindigkeiten für die Laminatbahn 1 bzw. 1' kann die Haftreibung zwischen Teillaminatbahn 5 und Auflagewalze 11 unzureichend für ein sicheres Vermeiden eines Schlupfs sein. Zur Verbesserung der Haftung der Teillaminatbahn 5 auf der Oberfläche der Auflagewalze 11 ist letztere bei bevorzugten Ausführungsformen als Vakuumwalze ausgebildet. Bei Ausführungsformen wie in Figur 2 veranschaulicht weist die Auflagewalze hierzu eine im Inneren angeordnete Saugkammer 111 auf, über die an der Auflagefläche für die Teillaminatbahn 5 ein Unterdruck erzeugt wird.

Unabhängig von der Ausbildung einer wie erläuterten Unterdrucckammer 111 in der Auflagewalze 11 wird das Ablösen der Teillaminatbahn 5 von der Auflagewalze 11 am Ende des Nips zur Kaschierwalze 15 durch Erzeugen eines Überdrucks an diesem Bereich der Walzenoberfläche unterstützt. Bei Ausführungsformen weist die Auflagewalze hierzu eine wie in Figur 2 veranschaulichte Druckkammer auf, die beidseits des Unterdruckbereichs einen Überdruck an der Walzenoberfläche schafft und somit bei sehr weichen Teillaminaten 5 verhindert, dass sich dieses beim Eintritt in den Nip zur Trennwalze 12 aufwellt. In der Regel genügt jedoch die Unterstützung des Ablösens der Teillaminatbahn von der Auflagewalze, so dass die Drucckammer bei Ausführungsformen auf einen wie beispielsweise in Figur 2 mithilfe der gepunkteten Linie angedeutet begrenzten Bereich beschränkt ist.

Bei von der in Figur 2 illustrierten Ausführungsform abweichenden Konstruktionen einer Schlaufenschlitzvorrichtung 10 sind entweder die Auftrenneinrichtung 12, die Umlenkeinrichtung 13 oder die Aufkaschiereinrichtung 15, bzw. zwei oder alle dieser Einrichtungen als Klingen ausgebildet. Unter Klinge ist hierbei ein Profil zu verstehen, das wenigstens eine Kante mit einer definierten Querschnittsgeometrie aufweist. Die Längsrichtung des Klingenprofils ist beim Einsatz in einer Schlaufenschlitzvorrichtung 10 quer zur Transportrichtung der Laminat- bzw. Folienbahn orientiert.

Bei Verwendung als Auftrenneinrichtung 12 weist die Klinge vorzugsweise einen rechteckförmigen Querschnitt mit einer an einem Eckbereich angeordneten Fase auf. Die Klinge liegt mit der Fase an der Fördereinrichtung 11 an und ist bei Ausführung der Fördereinrichtung als Walze bevorzugt dem Walzenradius angepasst gekrümmt profiliert, um hohe, auf die Laminatbahn einwirkende Druckkräfte zu vermeiden. Das Abziehen der Folienbahn 5 erfolgt an der bezüglich der Transportrichtung der Laminatbahn 1 hinteren Kante der Fase.

Auch die Aufkaschiereinrichtung kann als mit einer Fase versehene Klinge ausgeführt sein, wobei die Fase zum Erzielen eines gleichmäßigen Aufkaschierens in der Regel breiter als die Fase der Auftrenneinrichtung ist. Während bei der Auftrenneinrichtung die Fase mit geringem Anpressdruck an der Fördereinrichtung anliegt, um den Auftrennvorgang nicht zu beeinträchtigen, erfolgt das Kaschieren an der Fase der Aufkaschiereinrichtung mit höherem Anpressdruck.

Bei Ausführungsformen einer Schlaufenschlitzvorrichtung 10 ist die Umlenkeinrichtung von einer Klinge gebildet, die vorzugsweise eine Keilform mit stark abgerundeter Spitze aufweist. Der Keilwinkel ist bei vorteilhaften Ausführungsformen an den Umlenkwinkel der Folienbahn 2 angepasst. Falls die Schneidvorrichtung 14 an der klingenförmigen Umlenkeinrichtung anliegt, weist diese vorzugsweise eine stehende Schneide auf, die entweder an der Oberfläche der Klinge anliegt oder zum Ermöglichen eines Scherenschnitts an einer Seitenwand einer in der Klinge ausgebildeten Vertiefung anliegt.

Statt einer walzenförmigen Fördereinrichtung 11, wie sie in Figur 2 gezeigt ist, kann auch eine bandförmige Fördereinrichtung verwendet werden. Die bandförmige Fördereinrichtung weist zweckmäßig eine Endlosbespannung auf, die über zwei und gegebenenfalls mehr Walzen umgelenkt umläuft. Im Bereich zwischen Auftrenn- und Aufkaschiereinrichtung kann die Endlosband-Fördereinrichtung ferner eine Unterdruckeinrichtung zum Erzielen eines besseren Anhaftens des Teillaminats an der hierzu vorzugsweise perforiert ausgebildeten Bespannung aufweisen. Ferner kann am zum Aufkaschieren benutzten Bereich eine Überdruckvorrichtung ähnlich der für eine walzenförmige Fördereinrichtung beschriebenen angebracht sein.

Zum Erhalt von sich überlappenden Schutzfolienteilen können im Bereich zwischen Schlitzvorrichtung 14 und Aufkaschiereinrichtung 15 Führungselemente angeordnet sein, die die einzelnen Trägerfolienstreifen der geschlitzten Trägerfolie 2' so gegeneinander verschieben, dass sie sich bei Erreichen der Aufkaschiereinrichtung im Bereich der Wirkstoffdepotschichtbahn 3 bzw. -bahnen 3 überlappen.

## Patentansprüche

1. Vorrichtung zum Schlitzen einer einen Teil einer Laminatbahn (1) bildenden Folienbahn (2), wobei die Vorrichtung (10) eine Fördereinrichtung (11), eine Auftrenneinrichtung (12), eine Aufkaschiereinrichtung (15), eine Umlenkeinrichtung (13) und eine Schlitzvorrichtung (14) aufweist,
**dadurch gekennzeichnet, dass**
sowohl die Auftrenneinrichtung (12) als auch die Aufkaschiereinrichtung (15) an der Fördereinrichtung (11) anliegen oder einen Abstand zu dieser aufweisen, der kleiner oder gleich der Dicke der Laminatbahn (1) ist, die Aufkaschiereinrichtung (15) zur Auftrenneinrichtung (12) beabstandet und bezüglich der Förderrichtung der Fördereinrichtung (11) hinter der Auftrenneinrichtung (12) angeordnet ist,
die Umlenkeinrichtung (13) bezüglich der Förderrichtung der Fördereinrichtung (11) zwischen der Auftrenneinrichtung (12) und der Aufkaschiereinrichtung (15) und in einem Abstand zur Fördereinrichtung (11) angeordnet ist, und
die Schlitzvorrichtung (14) zum Einbringen eines Schlitzes in die von dem Laminat (1) getrennte Folienbahn (2) ausgebildet und im Bereich der Folienbahnführung zwischen Auftrenneinrichtung (12) und Aufkaschiereinrichtung (15) angeordnet ist,
sodass die Folienbahn (2) an der Auftrenneinrichtung (12) von der Laminatbahn (1) getrennt und über die Umlenkeinrichtung (13) der Aufkaschiereinrichtung (15) zugeführt werden kann, während die den anderen Teil der Laminatbahn (1) bildende Teillaminatbahn (5) mit gleicher Geschwindigkeit an der Fördereinrichtung (11) anliegend zur Aufkaschiereinrichtung (15) geführt werden kann, und ein Schlitzen der Folienbahn (2) im Bereich der Folienbahnführung zwischen Auftrenneinrichtung (12) und Aufkaschiereinrichtung (15) erfolgen kann.

2. Vorrichtung nach Anspruch 1, worin die Fördereinrichtung (11) von einer Walze gebildet ist.

3. Vorrichtung nach Anspruch 2, worin die Walze (11) zum Erzeugen eines Unterdrucks an zumindest dem von der Teillaminatbahn (5) umschlungenen Bereich ihrer Mantelfläche ausgebildet ist.

4. Vorrichtung nach Anspruch 2 oder 3, worin die Walze (11) zum Erzeugen eines Überdrucks an zumindest dem Bereich ausgebildet ist, der an den bezüglich der Förderrichtung hinteren Rand des von der Teillaminatbahn (5) umschlungenen Bereichs der Mantelfläche der Walze anschließt.

5. Vorrichtung nach einem der vorhergehenden Ansprüche, worin die Auftrenneinrichtung (12) und /oder die Aufkaschiereinrichtung (15) und/oder die Umlenkeinrichtung (13) als Walze ausgebildet ist.

6. Vorrichtung nach einem der vorhergehenden Ansprüche, worin die Auftrenneinrichtung (12) und /oder die Aufkaschiereinrichtung (15) und/oder die Umlenkeinrichtung (13) in Form einer Klinge ausgebildet ist.

7. Vorrichtung nach einem der vorhergehenden Ansprüche, worin die Umlenkeinrichtung (13) in einem Abstand zur Auftrenneinrichtung (12) und zur Aufkaschiereinrichtung (15) angeordnet ist.

8. Vorrichtung nach einem der vorhergehenden Ansprüche, worin die Umlenkeinrichtung (13) so gelagert ist, dass sie auf die um sie geführte Folienbahn (2) eine Zugspannung ausübt.

9. Vorrichtung nach einem der vorhergehenden Ansprüche, worin die Schlitzvorrichtung (14) einen Rotationskörper mit einer oder mehreren Schneiden umfasst, von denen jede entlang einer in Umfangsrichtung des Rotationskörpers geschlossen Linie angeordnet und entweder von einem Abschnitt oder von mehreren entlang der Linie zueinander beabstandeten Abschnitten gebildet ist.

10. Vorrichtung nach Anspruch 9, worin die Schnittkante der Schneide kreisförmig ausgebildet ist oder Abschnitte mit sich in Richtung der Rotationsachse des Rotationskörpers erstreckenden Komponenten aufweist.

11. Vorrichtung nach einem der Ansprüche 1 bis 9, worin die Schlitzvorrichtung (14) eine stehende Klinge mit einer Schneide aufweist.

12. Vorrichtung nach einem der Ansprüche 9 bis 11, worin die Schneide die Umlenkeinrichtung (13) am von der Folienbahn (2) umschlungenen Bereich berührt.

13. Vorrichtung nach Anspruch 10 oder 11, worin die Schneide, soweit kreisförmig oder stehend ausgebildet, in eine am Umschlingungsbereich der Umlenkeinrichtung (13) ausgebildete Nut eingreift.

14. Vorrichtung nach einem der Ansprüche 1bis 13, worin die Schlitzvorrichtung (14) als zwischen Auftrenneinrichtung (12) und Umlenkeinrichtung (13) und/oder zwischen Umlenkeinrichtung (13) und Aufkaschiereinrichtung (15) angeordnete Stanzvorrichtung, vorzugsweise als Hubstanze, ausgebildet ist.

15. Verfahren zum Schlitzen einer einen Teil einer Laminatbahn (1) bildenden Folienbahn (2), wobei das Verfahren auf einer Vorrichtung nach einem der vorhergehenden Ansprüche ausgeführt wird und Schritte umfasst zum
- Trennen der Folienbahn (2) von der restlichen Teilbahn (5) der Laminatbahn (1) an der Auftrenneinrichtung (12),
- Führen der abgetrennten Folienbahn (2) von der Auftrenneinrichtung (12) über die Umlenkeinrichtung (13) zur Aufkaschiereinrichtung (15),
- Schlitzen der Folienbahn (2) im Bereich zwischen Auftrenneinrichtung (12) und Aufkaschiereinrichtung (15),
- Führen der restlichen Teilbahn (5) der Laminatbahn (1) an der Fördereinrichtung (11) anliegend von der Auftrenneinrichtung (12) zur Aufkaschiereinrichtung (15),
wobei die Teilbahn (5) und die Folienbahn (2) mit gleicher Geschwindigkeit von der Auftrenneinrichtung (12) zur Aufkaschiereinrichtung (15) transportiert werden.

## Claims

1. An apparatus for slitting a film web (2) forming part of a laminate web (1), the apparatus (10) comprising:
a conveying device (11), a separating device (12), a laminating device (15), a guiding device (13), and a slitting device (14),
**characterized in that**:
both the separating device (12) and the laminating device (15) are in contact with the conveying device (11) or are located at a distance from the conveying device (11), which is less than or equal to the thickness of the laminate web (1), the laminating device (15) is located at a distance from the separating device (12) and, with respect to the conveying direction of the conveying device (11), disposed behind the separating device (12),
the guiding device (13) is, with respect to the conveying direction of the conveying device (11), disposed between the separating device (12) and the laminating device (15) and located at a distance from the conveying device (11), the slitting device (14) is configured for making a slit into the film web (2) separated from the laminate (1) and disposed in the film web routeing region between the separating device (12) and the laminating device (15),
thus enabling the film web (2) to be separated from the laminate web (1) at the separating device (12) and routed to the laminating device (15) via the guiding device (13), while the part-laminate web (5) forming the other part of the laminate web (1) is routed lying against the conveying device (11) at the same speed to the laminating device (15), with the slitting of the film web (2) taking place in the film web routeing region between the separating device (12) and the laminating device (15).

2. The apparatus according to claim 1, wherein the conveying device (11) is a roll.

3. The apparatus according to claim 2, wherein the roll (11) is configured to generate a vacuum in at least the region of its lateral surface looped around by the part-laminate web (5).

4. The apparatus according to claim 2 or 3, wherein the roll (11) is configured to generate an overpressure in at least the region adjacent to the, with respect to the conveying direction, rear edge of the lateral surface's region looped around by the part-laminate (5).

5. The apparatus according to any of the preceding claims, wherein at least one of the separating device (12), the laminating device (15), and the guiding device (13) is formed by a roll.

6. The apparatus according to any of the preceding claims, wherein at least one of the separating device (12), the laminating device (15), and the guiding device (13) is formed by a blade.

7. The apparatus according to any of the preceding claims, wherein the guiding device (13) is disposed at a distance to the separating device (12) and to the laminating device (15).

8. The apparatus according to any of the preceding claims, wherein the guiding device (13) is mounted to exert a tension on the film web (2) looped around it.

9. The apparatus according to any of the preceding claims, wherein the slitting device (14) comprises a rotary body having one or more blades, each of which is arranged along a line closed in the circumferential direction of the rotary body and formed by either one section or by a plurality of sections spaced apart along the line.

10. The apparatus according to claim 9, wherein the cutting edge of the blade is circular or comprises sections having components extending in the direction of the axis of rotation of the rotary body.

11. The apparatus according to any of claims 1 to 9, wherein the slitting device (14) comprises a stationary blade having a cutting edge.

12. The apparatus according to any of claims 9 to 11, wherein the blade contacts the guiding device (13) at the region looped around by the film web (2).

13. The apparatus according to claim 10 or 11, wherein the blade, provided that it is circular or stationary, engages in a groove formed on the looped-around region of the guiding device (13).

14. The apparatus according to any of claims 1 to 13, wherein the slitting device (14) is a punching device, preferably a lifting punch, disposed between the separating device (12) and the guiding device (13) and/or between the guiding device (13) and the laminating device (15).

15. A method for slitting a film web (2) forming part of a laminate web (1), the method being carried out using an apparatus according to any of the preceding claims and comprising the following steps:
- separating the film web (2) from the remaining part-laminate web (5) of the laminate web (1) at the separating device (12),
- routeing the separated film web (2) from the separating device (12) to the laminating device (15) via the guiding device (13),
- slitting the film web (2) in the region between the separating device (12) and the laminating device (15),
- routeing the remaining part-laminate web (5) of the laminate web (1) lying against the conveying device (11) from the separating device (12) to the laminating device (15),
wherein the part-laminate web (5) and the film web (2) are conveyed from the separating device (12) to the laminating device (15) at the same speed.

## Revendications

1. Dispositif d'incision d'une bande de film (2) formant une partie d'une bande laminée (1), où le dispositif (10) comporte un convoyeur (11), un dispositif de séparation (12), un dispositif de contrecollage (15), un dispositif de renvoi (13) et un dispositif d'incision (14), **caractérisé en ce qu'**aussi bien le dispositif de séparation (12) que le dispositif de contrecollage (15) sont placés contre le convoyeur (11) ou à une distance de celui-ci qui est inférieure ou égale à l'épaisseur de la bande laminée (1), le dispositif de contrecollage (15) est disposé à distance du dispositif de séparation (12) derrière le dispositif de séparation (12) par rapport au sens de déplacement du convoyeur (11),
le dispositif de renvoi (13) est disposé entre le dispositif de séparation (12) et le dispositif de contrecollage (15) par rapport au sens de déplacement du convoyeur (11) et à distance du convoyeur (11) et
le dispositif d'incision (14) est conçu pour introduire une incision dans la bande de film (2) séparée du laminé (1) et est disposé dans la zone d'acheminement de la bande de film entre le dispositif de séparation (12) et le dispositif de contrecollage (15) de telle sorte que la bande de film (2) puisse être séparée de la bande laminée (1) sur le dispositif de séparation (12) et être acheminée vers le dispositif de contrecollage (15) par l'intermédiaire du dispositif de renvoi (13), alors que la bande partielle laminée (5) formant l'autre part de la bande laminée (1) puisse être acheminée à la même vitesse vers le dispositif de contrecollage (15) en étant contre le convoyeur (11) et qu'une incision de la bande de film (2) puisse avoir lieu dans la zone d'acheminement de la bande de film entre le dispositif de séparation (12) et le dispositif de contrecollage (15).

2. Dispositif selon la revendication 1, dans lequel le convoyeur (11) est formé par un rouleau.

3. Dispositif selon la revendication 2, dans lequel le rouleau (11) est conçu pour engendrer une dépression sur au moins la zone de sa surface enveloppante entourée par la bande partielle laminée (5).

4. Dispositif selon la revendication 2 ou 3, dans lequel le rouleau (11) est conçu pour engendrer une surpression sur au moins la zone adjacente au bord arrière de la zone de la surface enveloppante du rouleau entourée par la bande partielle laminée (5) par rapport au sens de déplacement.

5. Dispositif selon l'une des revendications précédentes, dans lequel le dispositif de séparation (12) et / ou le dispositif de contrecollage (15) et / ou le dispositif de renvoi (13) sont réalisés sous forme d'un rouleau.

6. Dispositif selon l'une des revendications précédentes, dans lequel le dispositif de séparation (12) et / ou le dispositif de contrecollage (15) et / ou le dispositif de renvoi (13) sont réalisés sous forme d'une lame.

7. Dispositif selon l'une des revendications précédentes, dans lequel le dispositif de renvoi (13) est disposé à distance du dispositif de séparation (12) et du dispositif de contrecollage (15).

8. Dispositif selon l'une des revendications précédentes, dans lequel le dispositif de renvoi (13) est disposé de manière à exercer une tension sur la bande de film (2) l'entourant.

9. Dispositif selon l'une des revendications précédentes, dans lequel le dispositif d'incision (14) comprend un solide de révolution avec un ou plusieurs tranchants, dont chacun est disposé le long d'une ligne fermée dans le sens circonférentiel du solide de révolution et est formé soit par une section, soit par plusieurs sections espacées les unes des autres le long de la ligne.

10. Dispositif selon la revendication 9, dans lequel le fil du tranchant est réalisé en forme circulaire ou comporte des sections avec des éléments s'étendant dans le sens de l'axe de rotation du solide de révolution.

11. Dispositif selon l'une des revendications de 1 à 9, dans lequel le dispositif d'incision (14) comporte une lame stationnaire avec un tranchant.

12. Dispositif selon l'une des revendications de 9 à 11, dans lequel le tranchant est en contact avec le dispositif de renvoi (13) dans la zone entourée par la bande de film (2).

13. Dispositif selon la revendication 10 ou 11, dans lequel le tranchant, pour autant qu'il soit réalisé en forme circulaire ou de façon stationnaire, s'engage dans une rainure formée dans la zone d'enlacement du dispositif de renvoi (13).

14. Dispositif selon l'une des revendications de 1 à 13, dans lequel le dispositif d'incision (14) est conçu comme presse à découper, de préférence comme presse à découper de levage, disposée entre le dispositif de séparation (12) et le dispositif de renvoi (13) et / ou entre le dispositif de renvoi (13) et le dispositif de contrecollage (15).

15. Procédé pour inciser une bande de film (2) formant une partie d'une bande laminée (1), le procédé étant effectué sur un dispositif selon l'une des revendications précédentes et comprenant des étapes pour
- la séparation de la bande de film (2) de la bande partielle (5) restante de la bande laminée (1) sur le dispositif de séparation (12),
- l'acheminement de la bande de film (2) séparée depuis le dispositif de séparation (12) vers le dispositif de contrecollage (15) par l'intermédiaire du dispositif de renvoi (13),
- l'incision de la bande de film (2) dans la zone entre le dispositif de séparation (12) et le dispositif de contrecollage (15),
- l'acheminement de la bande partielle (5) restante de la bande laminée (1) en étant contre le convoyeur (11) depuis le dispositif de séparation (12) vers le dispositif de contrecollage (15),
dans lequel la bande partielle (5) et la bande de film (2) sont transportées à la même vitesse depuis le dispositif de séparation (12) vers le dispositif de contrecollage (15).
